# EUROPÄISCHE PATENTSCHRIFT

(11) **EP 1 470 788 B1**
(45) Veröffentlichungstag und Bekanntmachung des Hinweises auf die Patenterteilung: **14.06.2006**
(21) Anmeldenummer: 03009440.3
(22) Anmeldetag: 25.04.2003
(51) Int. Cl.: A61B 17/15

(54) **Vorrichtung zur Festlegung von Knochenschnitten**
Bone cutting guide
Dispositif de guidage pour coupes osseuses

(43) Veröffentlichungstag der Anmeldung: 27.10.2004
(73) Patentinhaber: Zimmer GmbH, 8404 Winterthur (CH)
(72) Erfinder: Faoro, Francisco, CH-8002 Zürich (CH); Overes, Tom, 8400 Winterthur (CH)
(74) Vertreter: Manitz, Finsterwald & Partner GbR

(56) Entgegenhaltungen:
- DE-U- 20 303 498
- FR-A- 2 679 766
- US-A- 4 349 018
- US-A- 4 759 350
- US-A- 5 342 367
- US-A- 5 364 402

## Beschreibung

Die Erfindung betrifft eine Vorrichtung zur Festlegung von Knochenschnitten beim Einsetzen von Knieimplantaten gemäß dem Oberbegriff des Anspruchs 1. Eine derartige Vorrichtung ist aus US 5,342,367 bekannt.

Beim Einsetzen von Knieprothesen müssen die Kondylen der Tibia und des Femur vorbereitet werden, um Anlageflächen an den Knochen zu schaffen, die eine definierte Lage der Tibia- und Femurimplantate der Knieprothese gewährleisten.

Die Anlageflächen werden durch Wegschneiden von Knochenmaterial an den Kondylen erzeugt. Dabei ist man bestrebt, so wenig Knochenmaterial wie möglich zu entfernen. Ferner muss darauf geachtet werden, dass die Schnittflächen an der Tibia und am Femur richtig relativ zueinander orientiert sind, damit die Tibia- und Femurimplantate in einer der natürlichen Bewegung des Kniegelenks entsprechenden Weise zusammenwirken können.

Schwierig ist dies insbesondere bei unikompartimentalen Operationen, bei denen entweder nur im medialen oder im lateralen Kompartiment eine Knieprothese implantiert werden soll. Bislang existieren keine Instrumente, mit denen die hierfür auszuführenden Knochenschnitte sowohl hinsichtlich ihrer Lage und Orientierung relativ zu der jeweiligen Kondyle als auch hinsichtlich der Schnitttiefe vor allem in der Richtung lateral-medial mit der gewünschten Genauigkeit festgelegt werden können.

US 5,364,402 betrifft eine Schnittlehre mit einer Platte für einen vertikalen Schnitt und einem Block für einen horizontalen Schnitt, wobei die Schnittlehre auf ein bereits reseziertes Tibiaplateau aufsetzbar ist, um die laterale oder mediale Kondyle der Tibia zu resezieren.

Aufgabe der Erfindung ist es, eine Vorrichtung der eingangs genannten Art zu schaffen, mit der die an der jeweiligen Kondyle auszuführenden Knochenschnitte auf möglichst einfache und zuverlässige Weise mit einer möglichst hohen Genauigkeit festgelegt werden können, wobei dies insbesondere unter möglichst weitgehender Berücksichtigung der jeweiligen anatomischen Verhältnisse erfolgen soll.

Die Lösung dieser Aufgabe erfolgt durch die Merkmale des Anspruchs 1 und insbesondere dadurch, dass die Schnittlehre im festgesetzten Zustand jeweils mittels eines Schlitzes für ein Schneidewerkzeug eine erste Schnittebene bezüglich des Basiselements sowie eine zweite Schnittebene bezüglich der ersten Schnittebene definiert, wobei die Orientierung der zweiten Schnittebene bei mit dem Basiselement gekoppelter Schnittlehre und unter Berücksichtigung der jeweiligen Knieanatomie relativ zur ersten Schnittebene einstellbar ist.

Vorzugsweise ist die erfindungsgemäße Vorrichtung zur Festlegung von Knochenschnitten an einer Tibiakondyle im Rahmen einer unikompartimentalen Knieoperation ausgebildet, durch die ein bevorzugt von zwei senkrecht zueinander verlaufenden Schnittflächen definiertes Tibiaplateau erzeugt wird, auf das im weiteren Verlauf der Operation ein Tibiaimplantat der Knieprothese aufgesetzt werden kann.

Im Folgenden wird bei der weiteren Erläuterung der Erfindung zur Vereinfachung der Darstellung ausschließlich auf deren Verwendung in Verbindung mit einer Tibiakondyle Bezug genommen.

Die Erfindung geht von einem hinsichtlich Lage und Orientierung korrekt im Bereich einer vorzubereitenden Kondyle fixierten Basiselement aus, welches lediglich eine Ebene definiert, ohne dass die Schnitttiefe in irgendeiner Richtung in der Ebene oder parallel zu der Ebene definiert ist.

Mit diesem Basiselement steht ein Bezug zur Verfügung, relativ zu welchem mittels des erfindungsgemäßen Instrumentariums die erste Schnittebene festgelegt wird. Erfindungsgemäß ist es möglich, bezüglich dieser ersten Schnittebene eine zweite Schnittebene zu definieren und die Orientierung dieser zweiten Schnittebene relativ zur ersten Schnittebene einzustellen. Dies kann erfindungsgemäß unter Berücksichtigung der jeweiligen Knieanatomie erfolgen. Folglich gestattet es die Erfindung, zwei Schnittebenen nicht nur gezielt in Bezug auf das Basiselement sowie relativ zueinander festzulegen, sondern dabei außerdem die anatomischen Gegebenheiten des jeweiligen Knies zu berücksichtigen.

Bevorzugte Ausführungsformen der Erfindung sind in den Unteransprüchen, der Beschreibung sowie der Zeichnung angegeben.

Es ist bevorzugt vorgesehen, dass die erste Schnittebene und die zweite Schnittebene senkrecht zueinander verlaufen.

Des Weiteren kann erfindungsgemäß vorgesehen sein, dass die Orientierung der zweiten Schnittebene bei relativ zum Basiselement bewegbarer Schnittlehre einstellbar ist. Hierdurch kann gleichzeitig die Schnittlehre relativ zu dem Basiselement bewegt und die Orientierung der zweiten Schnittebene verändert werden, wodurch die an der Schnittlehre ausgebildeten Schlitze optimal entsprechend den jeweiligen anatomischen Gegebenheiten des Knies ausgerichtet werden können, bevor die Schnittlehre am Basiselement festgesetzt wird.

Vorzugsweise ist die Schnittlehre im noch nicht festgesetzten Zustand ausschließlich parallel zur ersten Schnittebene bewegbar. Die Lage und die Orientierung der ersten Schnittebene relativ zu dem Basiselement ändern sich hierdurch beim Bewegen der Schnittlehre relativ zu dem Basiselement nicht. Ferner kann vorgesehen sein, dass die Schnittlehre ausschließlich geradlinig relativ zu dem Basiselement bewegbar ist. Hierzu kann am Basiselement eine Führungsnut ausgebildet sein, durch welche die Schnittlehre am Basiselement zwangsgeführt ist.

Gemäß einem weiteren Ausführungsbeispiel der Erfindung wird vorgeschlagen, dass die Schnittlehre wenigstens eine Führung für ein Anschlagelement aufweist, das in zumindest einer der Schnittebenen einen den betreffenden Schnitt begrenzenden Anschlag für das Schneidewerkzeug bildet.

Hierdurch kann auf einfache und sichere Weise die Schnitttiefe in der entsprechenden Richtung begrenzt werden, so dass eine ungewollte Schädigung des Knochens vermieden wird. Der Operateur kann sich auf die Ausführung des Schnittes konzentrieren und braucht nicht auf die Schnitttiefe zu achten, da er sich diesbezüglich auf das korrekt positionierte Anschlagelement verlassen kann.

Die Führung für das Anschlagelement kann derart ausgebildet sein, dass das Anschlagelement auf der Schnittlinie der beiden Schnittebenen liegt. Hierdurch kann mittels des Anschlagelementes die gewünschte Schnittlinie der beiden Schnittebenen exakt festgelegt werden. Dabei wird mit nur einem einzigen Anschlagelement eine Begrenzung für beide Schnittebenen festgelegt.

Ferner kann erfindungsgemäß vorgesehen sein, dass die Führung für das Anschlagelement mit dem die zweite Schnittebene definierenden Schlitz zwangsgekoppelt ist. Hierdurch wird beim Einstellen der Orientierung der zweiten Schnittebene gleichzeitig die Orientierung des Anschlagelementes festgelegt, wodurch die Handhabung des erfindungsgemäßen Instrumentes weiter vereinfacht wird.

Die Führung kann einen in der Schnittlehre ausgebildeten Durchgang umfassen, durch den das Anschlagelement hindurchsteckbar ist. Dieser Durchgang kann gleichzeitig als Führung für einen Bohrer dienen, mit dem der Knochen zunächst vorgebohrt wird, um anschließend das Anschlagelement in den Knochen einführen zu können.

In einer bevorzugten Ausführungsform der Erfindung ist zur Berücksichtigung der Knieanatomie eine verstellbare Tasteinrichtung vorgesehen, die mit dem die zweite Schnittebene definierenden Schlitz der Schnittlehre zwangskoppelbar und an der durch Verstellen eines Tasters relativ zu einem mit dem Schlitz gekoppelten Grundteil eine Mehrzahl vorgegebener Implantatgrößen einstellbar ist.

Zur Koppelung mit dem Schlitz kann ein Kopplungsabschnitt vorgesehen sein, über den die Tasteinrichtung die Lage von Eminentia und Kreuzbandverankerung antasten kann, um den die zweite Schnittebene definierenden Schlitz mit Sicherheitsabstand fixierbar zu machen.

Mit einer derartigen Tasteinrichtung kann die Orientierung der zweiten Schnittebene in Abhängigkeit von den anatomischen Gegebenheiten des jeweiligen Knies eingestellt werden. Der Operateur kann durch Verändern der Orientierung der zweiten Schnittebene unter gleichzeitigem Verstellen des Tasters relativ zu dem mit dem Schlitz gekoppelten Grundteil die für die Operation am besten geeignete Implantatgröße ermitteln.

Erfindungsgemäß ist insbesondere vorgesehen, dass der die erste Schnittebene definierende Schlitz von einem bei mit dem Basiselement gekoppelter Schnittlehre zwischen der Oberseite des Basiselementes und einer dem Basiselement zugewandten Seite der Schnittlehre vorhandenen Zwischenraum gebildet ist. Beim Ausführen des ersten Schnittes wird das Schneidewerkzeug folglich zwischen dem Basiselement und der Schnittlehre geführt. Dabei kann das Schneidewerkzeug auf der Oberseite des Basiselementes aufliegen. Eine Verschiebung der Schnittlehre relativ zu dem Basiselement ändert an der Orientierung und der Lage des die erste Schnittebene definierenden Schlitzes nichts.

In einem weiteren Ausführungsbeispiel der Erfindung ist vorgesehen, dass die Schnittlehre eine Drehtelleranordnung mit einem an der Schnittlehre drehbar gelagerten Drehteller umfasst, mit dem ein Führungsabschnitt drehfest verbunden ist, in dem der die zweite Schnittebene definierende Schlitz ausgebildet ist. Dabei kann die Drehachse des Drehtellers senkrecht zur ersten Schnittebene verlaufen.

Ferner kann vorgesehen sein, dass mit dem Drehteller ein weiterer Führungsabschnitt drehfest verbunden ist, der als Führung für das Anschlagelement ausgebildet ist. Hierdurch kann auf einfache Weise die Relativlage zwischen dem die zweite Schnittebene definierenden Schlitz und der Führung für das Anschlagelement fest vorgegeben werden.

Die Schnittlehre kann des Weiteren eine Klemmeinrichtung mit einem Betätigungsorgan aufweisen, mittels welchem gleichzeitig die Schnittlehre relativ zum Basiselement und die Orientierung der zweiten Schnittebene relativ zur ersten Schnittebene festgesetzt werden kann. Gewissermaßen mit einem einzigen Handgriff kann hierdurch die erfindungsgemäße Schnittlehre gleichzeitig hinsichtlich aller zur Festlegung der Knochenschnitte vorgesehener Bewegungsfreiheitsgrade in der gewünschten Stellung fixiert werden.

Die Schnittlehre kann ein U-förmig ausgebildetes, auf das Basiselement aufschiebbares Grundteil mit parallel zur ersten Schnittebene verlaufenden U-Schenkeln, einen um eine parallel zur ersten Schnittebene und senkrecht zu den U-Schenkeln des Grundteils verlaufende Achse schwenkbar am Grundteil gelagerten Klemmhebel und eine sich parallel zu den U-Schenkeln des Grundteils erstreckende, mit dem Klemmhebel über ein Gewinde zusammenwirkende Klemmspindel umfassen, wobei durch eine Drehbetätigung der Klemmspindel gleichzeitig eine am oberen U-Schenkel des Grundteils um eine senkrecht zur ersten Schnittebene verlaufende Achse drehbar gelagerte, den die zweite Schnittebene definierenden Schlitz aufweisende Drehtelleranordnung festsetzbar und der Klemmhebel relativ zum Grundteil in Klemmeingriff mit dem Basiselement schwenkbar ist.

Dabei kann ein freier Endbereich der Klemmspindel zum Festsetzen der Drehtelleranordnung ausgebildet und durch die Drehbetätigung der Klemmspindel in Klemmeingriff mit einem äußeren Randbereich der Drehtelleranordnung bringbar sein.

Ferner kann der Klemmhebel einen Betätigungsarm und einen Klemmarm umfassen, wobei die Klemmspindel mit dem Betätigungsarm zusammenwirkt und der Klemmarm zum Festklemmen des zwischen den beiden U-Schenkeln des Grundteils befindlichen Basiselementes durch die Drehbetätigung der Klemmspindel über den Betätigungsarm in den Bereich zwischen den beiden U-Schenkeln hinein und gegen das Basiselement schwenkbar ist.

Ein versehentliches Lösen der Klemmung, beispielsweise durch während des Ausführens der Knochenschnitte auftretende Vibrationen, kann auf besonders sichere Weise verhindert werden, wenn gemäß einer weiteren Ausführungsform der Erfindung zusammenwirkende Gewindeabschnitte der Klemmspindel und des Klemmhebels durch eine zwischen einen spindelfesten Auflageabschnitt und den Betätigungsarm des Klemmhebels gespannte Feder spielfrei gehalten sind.

Vorzugsweise ist der Zwischenraum zwischen den beiden U-Schenkeln des Grundteils auf die Höhe des Basiselementes abgestimmt.

Die Erfindung ermöglicht die Verwendung vergleichsweise dünner Basiselemente, die beispielsweise eine Dicke von lediglich etwa 12 mm aufweisen.

Die Erfindung wird im Folgenden beispielhaft unter Bezugnahme auf die Zeichnung beschrieben. Es zeigen:
- Fig. 1 bis 5: verschiedene Phasen eines Teils einer Knieoperation, bei dem mittels einer erfindungsgemäßen Vorrichtung Knochenschnitte an einer Tibiakondyle ausgeführt werden,
- Fig. 6: verschiedene Ansichten einer Schnittlehre gemäß einer Ausführungsform der Erfindung,
- Fig. 7: verschiedene Ansichten einer mit der Schnittlehre von Fig. 6 verwendbaren Tasteinrichtung gemäß einer Ausführungsform der Erfindung, und
- Fig. 8: verschiedene Ansichten eines mit der Schnittlehre von Fig. 6 verwendbaren Anschlagelementes gemäß einer Ausführungsform der Erfindung.

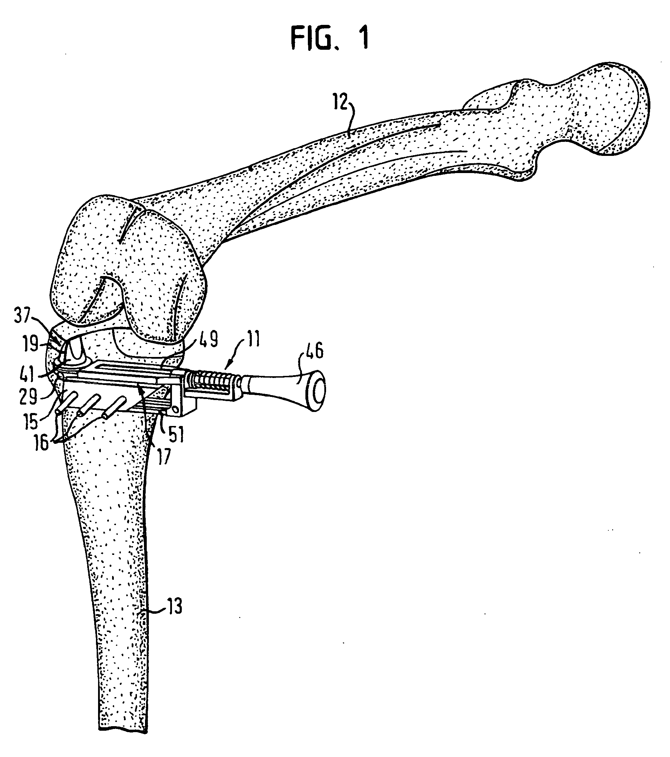

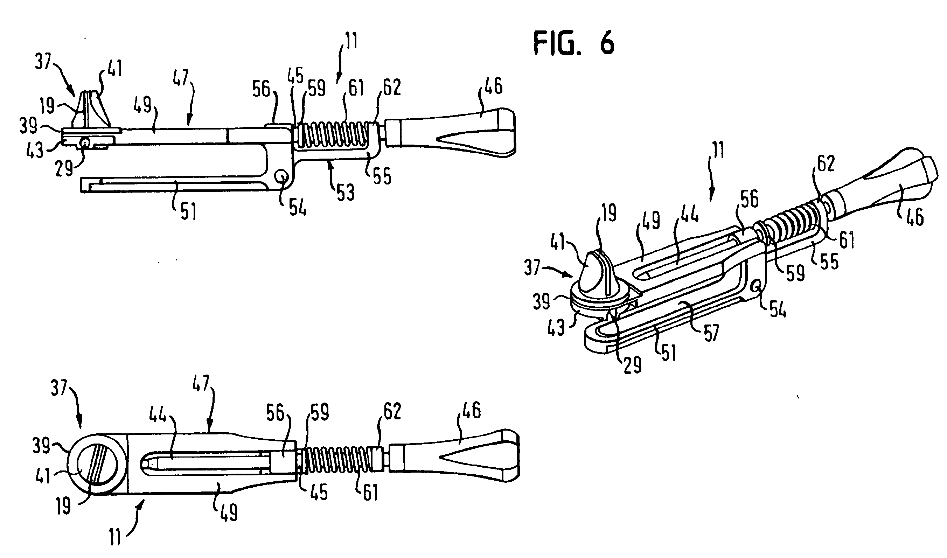

Bevor anhand der Fig. 6 bis 8 auf konstruktive Details der erfindungsgemäßen Vorrichtung eingegangen wird, soll zunächst anhand der Fig. 1 bis 5 derjenige Teil einer Knieoperation erläutert werden, bei dem die erfindungsgemäße Vorrichtung zum Einsatz kommt.

Ziel der Gesamt-Operation ist es, eine Knieprothese bestehend aus einem Tibiaimplantat und einem Femurimplantat einzusetzen. In Verbindung mit der Erfindung sind vor allem unikompartimentale Operationstechniken von Interesse, durch die zunächst für das einzusetzende Tibiaimplantat an der betreffenden Tibiakondyle durch Wegschneiden von Knochenmaterial ein Plateau geschaffen wird, woraufhin die gegenüberliegende Femurkondyle ebenfalls durch Wegschneiden von Knochenmaterial für die Fixierung eines entsprechenden Femurimplantats vorbereitet wird.

Hierbei kommt es nicht nur auf eine korrekte Positionierung des Tibiaimplantats an der Tibia und des Femurimplantats an dem Femur, sondern ganz entscheidend auch auf die richtige Ausrichtung des Tibiaimplantats und des Femurimplantats relativ zueinander an. Um die richtige Positionierung der Implantate zu gewährleisten, ist neben einer guten Operationsplanung eine exakte Ausführung der zum Entfernen des Knochenmaterials erforderlichen Knochenschnitte von entscheidender Bedeutung.

Die vorliegende Erfindung betrifft gemäß der in den Figuren dargestellten Ausführungsform die Festlegung der an einer Tibiakondyle zur Herstellung eines Plateaus für ein Tibiaimplantat auszuführenden Knochenschnitte mittels eines Instrumentariums, welches es gestattet, die Schnittebenen unter Berücksichtigung der anatomischen Gegebenheiten des jeweiligen Knies festzulegen, bevor die Schnitte mittels insbesondere in Form von Sägen vorgesehener Schneidewerkzeuge ausgeführt werden.

Ausgangspunkt für den mittels des erfindungsgemäßen Instrumentariums durchführbaren Teil der Gesamt-Operation ist ein an der Tibia 13 mittels Haltestiften 16 fixiertes, im Folgenden auch als Schnittblock bezeichnetes Basiselement 15. Das Basiselement 15 wurde zuvor derart relativ zur Tibia 13 positioniert, dass die ebene Oberseite des Basiselementes 15 auf der Höhe des gemäß der Operationsplanung durch die festzulegenden Knochenschnitte herzustellenden Tibiaplateaus liegt. Mit anderen Worten: Die Oberseite des Schnittblocks 15 gibt Lage und Orientierung derjenigen Schnittebene relativ zur Tibia 13 vor, die im Folgenden auch als erste Schnittebene bezeichnet wird.

Damit ist dem Operateur aber noch nicht bekannt, mit welcher Schnitttiefe dieser erste Knochenschnitt in der Richtung lateral-medial auszuführen ist. Ebenso wenig ist dem Operateur bereits bekannt, wie der das Tibiaplateau nach innen begrenzende zweite Knochenschnitt relativ zur Tibia und damit zur ersten Schnittebene zu orientieren ist. Sowohl die optimale Tiefe des ersten Schnittes in Richtung auf die Kreuzbänder und die Eminentia als auch die optimale Orientierung des zweites Schnittes entlang der Kreuzbänder und der Eminentia sind von der jeweiligen Knieanatomie abhängig und mussten bislang vom Operateur während der Operation ohne jegliche Hilfsmittel und damit ausschließlich "nach Gefühl" festgelegt werden.

Hier setzt die Erfindung an. Zunächst wird auf den Schnittblock 15 eine Schnittlehre 11 mit einem U-förmigen Grundteil auf den Schnittblock 15 geschoben. Das Grundteil umfasst einen oberen U-Schenkel 49 und einen unteren U-Schenkel 51. Mit dem unteren U-Schenkel 51 ist die Schnittlehre 11 in einer an der Unterseite des Schnittblocks 15 ausgebildeten, beispielsweise T-förmigen Nut derart geführt, dass die Schnittlehre 11 nur in der durch die Oberseite des Schnittblocks 15 festgelegten Ebene und parallel zu der Führungsnut des Schnittblocks 15, d.h. parallel zu den U-Schenkeln 49, 51, relativ zum Schnittblock 15 bewegt werden kann.

Mittels eines schlitzförmigen Zwischenraumes 17 zwischen dem oberen U-Schenkel 49 und der Oberseite des Basiselementes 15 definiert die Schnittlehre 11 eine erste Schnittebene für einen ersten Knochenschnitt. Dabei dient der Schlitz 17 als Führung für das zur Ausführung dieses Horizontalschnittes vorgesehene, als Sägeblatt ausgebildete Schneidewerkzeug.

Ein senkrecht zu dem ersten Schlitz 17 verlaufender zweiter Schlitz 19 ist in einer Drehtelleranordnung 37 der Schnittlehre 11 ausgebildet. Die Drehtelleranordnung 37 gestattet es, die Orientierung des zweiten Schlitzes 19 relativ zu der durch den ersten Schlitz 17 definierten Schnittebene einzustellen.

Eine mit einem Handgriff 46 versehene Gewindespindel 45 ermöglicht es, gleichzeitig die Drehtelleranordnung 37 in der jeweils eingestellten Orientierung zu fixieren und die Schnittlehre 11 am Basiselement 15 festzuklemmen. Dieses Festsetzen der Schnittlehre 11 und der Drehtelleranordnung 37 erfolgt, wenn die optimale Orientierung der durch den zweiten Schlitz 19 festgelegten Schnittebene gefunden ist.

Um die optimale Orientierung der zweiten vertikalen Schnittebene zu finden, wird mit der Schnittlehre 11 eine Tasteinrichtung 81 gekoppelt. Die Tasteinrichtung 81 weist ein Grundteil 85 mit einem streifenförmigen Kopplungsabschnitt 93 auf, der in den Schlitz 19 der Drehtelleranordnung 37 eingeführt wird, um eine Zwangskoppelung zwischen der Tasteinrichtung 81 und dem die zweite Schnittebene definierenden Schlitz 19 herzustellen. Über den Kopplungsabschnitt 93 des Grundteils 85 kann folglich die Tasteinrichtung 81 als Ganzes relativ zu der Schnittlehre 11 und damit relativ zu der Tibia 13 um eine senkrecht zu der durch den ersten Schlitz 17 definierten Schnittebene verlaufende Achse gedreht werden.

Ein weiterer Bewegungsfreiheitsgrad der Tasteinrichtung 81 ist durch einen einen Betätigungsabschnitt 87 und einen Tastarm 89 umfassenden Taster gegeben, der auf zwei fest mit dem Grundteil 85 verbundenen Führungsstiften 91 parallel zur ersten Schnittebene längsverschieblich geführt ist. Der Taster 83 kann in verschiedenen Stellungen, die jeweils einer vorgegebenen Implantatgröße entsprechen, an einem der Führungsstifte 91 verrastet werden.

Nach dem Aufschieben der Schnittlehre 11 auf den Schnittblock 15 und dem Einführen des Kopplungsabschnitts 93 in den drehbaren Schlitz 19 der Schnittlehre 11 kann die optimale Orientierung der durch den Schlitz 19 festgelegten zweiten Schnittebene durch Verschieben des Kopplungsabschnitts 93 in medio-lateraler Richtung und durch Verdrehen der Tasteinrichtung 81 relativ zur Schnittlehre 11 ertastet und mit dem Handgriff festgesetzt werden. In einem weiteren Schritt kann durch Verschieben des Tasters 83 längs der Führungsstifte 91 und durch Bewegen der Tasteinrichtung längs der zweiten Schnittebene vom Operateur die richtige Größe des Implantats relativ zur Außenkante an der Tibia bestimmt werden. Bei einer so bestimmten Implantatgröße kann durch kurzzeitiges Lösen und erneutes Anziehen des Handgriffs 46 jederzeit noch eine Feinkorrektur durchgeführt werden.

Dabei kann der Operateur mit der einen Hand den Betätigungsabschnitt 87 der Tasteinrichtung 81 und mit der anderen Hand den Handgriff 46 der Schnittlehre 11 ergreifen und so die Gesamtanordnung aus Schnittlehre 11 und Tasteinrichtung 81 entsprechend der gegebenen Bewegungsfreiheitsgrade in eine die anatomischen Gegebenheiten des jeweiligen Knies optimal berücksichtigende Konfiguration bringen.

Die optimale Konfiguration ermittelt der Operateur durch Ertasten mittels des Kopplungsabschnitts 93 und des Tastarms 89 sowie durch visuelles Überprüfen der Lage des Kopplungsabschnitts 93 sowie des Tastarms 89 relativ zu der Tibia 13. Zur Erleichterung der visuellen Überprüfung ist das freie Ende des Tastarms 89 entsprechend der äußeren seitlichen Begrenzung der späteren Implantate gekrümmt ausgebildet.

Wenn die optimale Konfiguration des erfindungsgemäßen Instrumentariums und damit die optimale Orientierung der zweiten Schnittebene sowie die am besten geeignete Implantatgröße eingestellt ist, werden durch Drehen der Gewindespindel über den Handgriff 46 die mit dem Schlitz 19 versehene Drehtelleranordnung 37 bezüglich der Schnittlehre 11 und die Schnittlehre 11 bezüglich des Schnittblocks 15 festgesetzt. Die Tasteinrichtung 81 wird dann abgenommen.

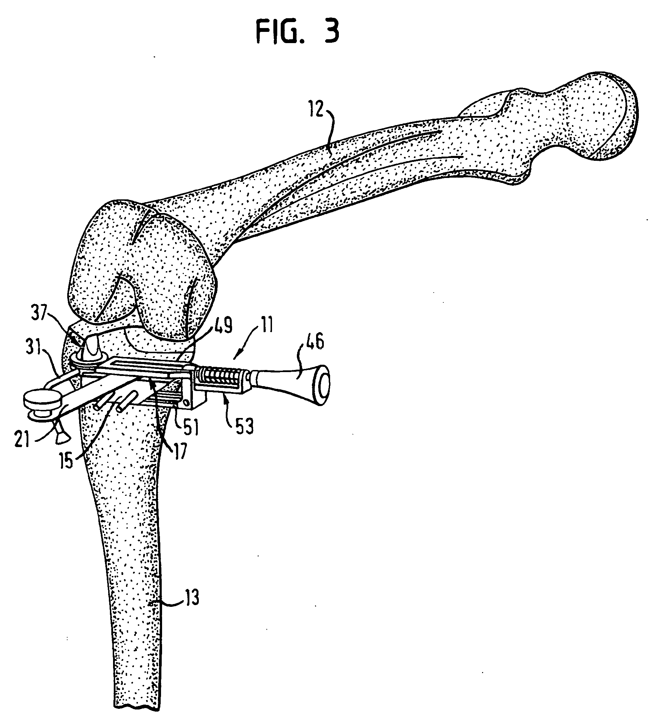

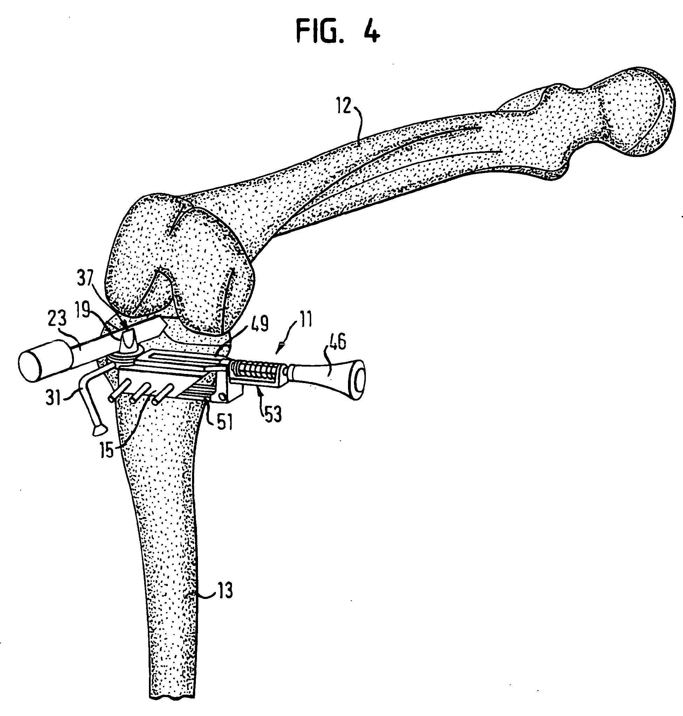

Anschließend wird - geführt über einen ebenfalls an der Drehtelleranordnung 37 ausgebildeten, mit dem Schlitz 19 ausgerichteten Durchgang 29 (vgl. Fig. 1) - eine Bohrung im Knochen ausgebildet, in die daraufhin ein im Durchgang 29 geführter Anschlagstift 31 gesteckt wird (vgl. Fig. 3). Der als Führung sowohl für das Bohrwerkzeug als auch für den Anschlagstift 31 dienende Durchgang 29 ist derart orientiert, dass seine Mittelachse parallel zu der gedachten Schnittlinie zwischen dem ersten Schlitz 17 und dem zweiten Schlitz 19 verläuft, und dass sein Längsschnitt möglichst vollständig die Fortsetzung der Schlitze 17, 19 abdeckt. Folglich dient der durch den Durchgang 29 in den Knochen eingeführte Anschlagstift 31 als sowohl den ersten Schnitt als auch den zweiten Schnitt seitlich begrenzender Anschlag für durch die Schlitze 17, 19 geführte Sägeblätter 21, 23 (vgl. Fig. 3 und 4).

Nach dem Einführen des Anschlagstiftes 31 in den Knochen wird zunächst gemäß Fig. 3 mittels des durch den ersten Schlitz 17 geführten Sägeblatts 21 der erste Schnitt ausgeführt, dessen Tiefe in der Richtung lateral-medial durch den Anschlagstift 31 begrenzt ist. Anschließend wird gemäß Fig. 4 mittels des durch den zweiten Schlitz 19 geführten Sägeblatts 23 ein senkrecht zum ersten Schnitt verlaufender zweiter Schnitt ausgeführt, dessen Tiefe in distaler Richtung ebenfalls durch den Anschlagstift 31 begrenzt ist.

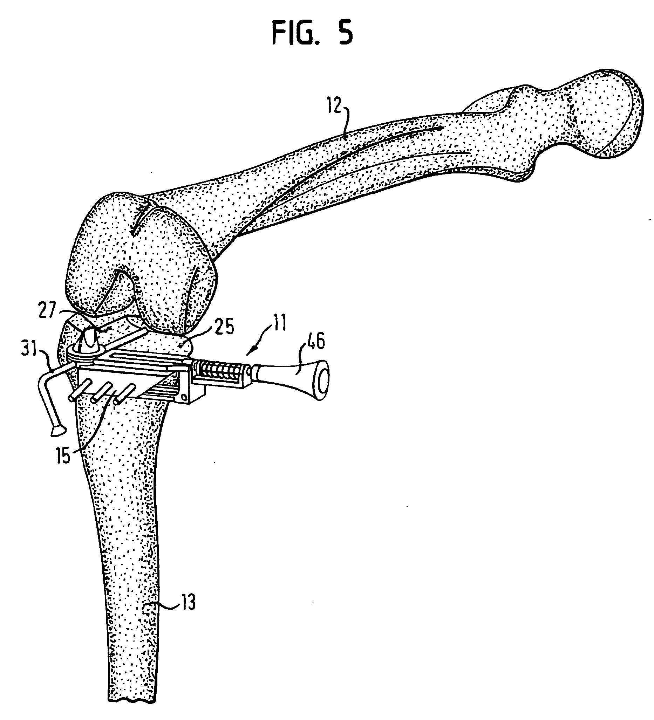

Fig. 5 zeigt das mittels des erfindungsgemäßen Instrumentariums geschaffene, durch senkrecht zueinander verlaufende Schnittflächen 25, 27 begrenzte Tibiaplateau, wobei außerdem der mit der Schnittlinie der beiden Schnittflächen 25, 27 zusammenfallende Anschlagstift 31 gezeigt ist.

Anschließend wird der Anschlagstift 31 herausgezogen, die Klemmung der Schnittlehre 11 am Schnittblock 15 aufgehoben und die Schnittlehre 11 vom Schnittblock 15 abgenommen. Damit ist die Tibia 13 zum Einsetzen des Tibiaimplantats der Knieprothese vorbereitet, wobei zuvor jedoch die gegenüberliegende Femurkondyle vorbereitet wird, worauf hier aber nicht näher eingegangen wird.

Gemäß Fig. 6 umfasst die erfindungsgemäße Schnittlehre 11 das U-förmige Grundteil 47 mit den beiden parallel verlaufenden U-Schenkeln 49, 51. Das freie Ende des oberen U-Schenkels 49 trägt die Drehtelleranordnung 37, die einen drehbar am oberen U-Schenkel 49 gelagerten Drehteller 39 sowie einen oberen, mit dem Schlitz 19 versehenen Führungsabschnitt 41 und einen unteren, mit dem Durchgang 29 versehenen unteren Führungsabschnitt 43 umfasst. Die Führungsabschnitte 41, 43 sind drehfest mit dem Drehteller 39 verbunden. Der Durchgang 29 ist mit dem Schlitz 19 ausgerichtet, d.h. die Mittelachse des Durchgangs 29 liegt in der durch den Schlitz 19 definierten zweiten Schnittebene.

An dem Grundteil 47 ist ein Klemmhebel 53 über einen Stift 54 um eine senkrecht zu den beiden U-Schenkel 49, 51 verlaufende Achse verschwenkbar gelagert. Der Klemmhebel 53 umfasst einen Klemmarm 57 und einen Betätigungsarm 55, die parallel versetzt zueinander verlaufen. Der zungenartig ausgebildete Klemmarm 57 erstreckt sich in einer entsprechend geformten Aussparung des unteren U-Schenkels 51 und ist durch Verschwenken des Klemmhebels 53 in den Bereich zwischen den beiden U-Schenkeln 49, 51 hinein verschwenkbar.

Der Klemmhebel 53 umfasst des Weiteren einen mit einem Innengewinde versehenen Gewindeabschnitt 56, der mit einer ein entsprechendes Außengewinde aufweisenden Klemmspindel 45 zusammenwirkt. Die Spindel 45 erstreckt sich in der einen Richtung durch eine Führungsöffnung hindurch, die in einem von dem freien Endbereich des Betätigungsarms 55 des Klemmhebels 53 gebildeten Abstütz- und Führungsabschnitt 62 ausgebildet ist. An ihrem freien Ende ist die Klemmspindel 45 mit dem Handgriff 46 versehen. In die entgegengesetzte Richtung erstreckt sich die Klemmspindel 45 in Form eines stiftförmigen Klemmabschnitts 44, dessen nicht dargestelltes freies Ende in Klemmeingriff mit einem äußeren Randbereich der Drehtelleranordnung 37 gebracht werden kann, wenn die Spindel 45 in die entsprechende Richtung gedreht wird.

Zwischen einem fest mit der Spindel 45 verbundenen Auflageabschnitt 59 und dem Abstütz- und Führungsabschnitt 62 ist eine Druckfeder 51 eingespannt, die das Außengewinde der Spindel 45 und das Innengewinde des Gewindeabschnitts 56 des Klemmhebels 53 in jeder Stellung der Spindel 45 spielfrei hält.

Durch Drehen der Spindel 45 wird das freie Ende des Klemmabschnitts 44 der Spindel 45 in Richtung der Drehtelleranordnung 37 und schließlich in Klemmeingriff mit der Drehtelleranordnung 37 bewegt, wodurch die Drehtelleranordnung 37 relativ zum Grundteil 47 festgesetzt wird. Sobald die Spindel 45 durch die Drehtelleranordnung 37 an einer axialen Weiterbewegung gehindert wird, führt ein Weiterdrehen der Spindel 45 zu einer axialen Bewegung des Gewindeabschnitts 56 des Klemmhebels 53 weg von der Drehtelleranordnung 37 und damit zu einem Verschwenken des Klemmhebels 53, was zur Folge hat, dass sich der Klemmarm 57 des Klemmhebels 53 in Richtung des oberen U-Schenkels 49 des Grundteils 47 und damit in den Zwischenraum zwischen den beiden U-Schenkeln 49, 51 hineinbewegt.

Wie vorstehend in Verbindung mit den Fig. 1 bis 5 bereits erläutert, erfolgt die Betätigung der die Klemmspindel 45 und den Klemmhebel 53 umfassenden Klemmeinrichtung bei auf den Schnittblock 15 geschobener Schnittlehre 11, d.h. in dem Zwischenraum zwischen den beiden U-Schenkeln 49, 51 befindet sich der Schnittblock 15. Daher reicht eine relativ kleine Schwenkbewegung des Klemmhebels 53 aus, um die Schnittlehre 11 über den mit der Unterseite des Schnittblocks 15 in Klemmeingriff gelangenden Klemmarm 57 am Schnittblock 15 festzusetzen.

Durch Betätigen der Spindel 45 über den Handgriff 46 kann somit gleichzeitig die Drehtelleranordnung 37 bezüglich des Grundteils 47 der Schnittlehre 11 und die Schnittlehre 11 bezüglich des Schnittblocks 15 sowohl fixiert als auch wieder gelöst werden.

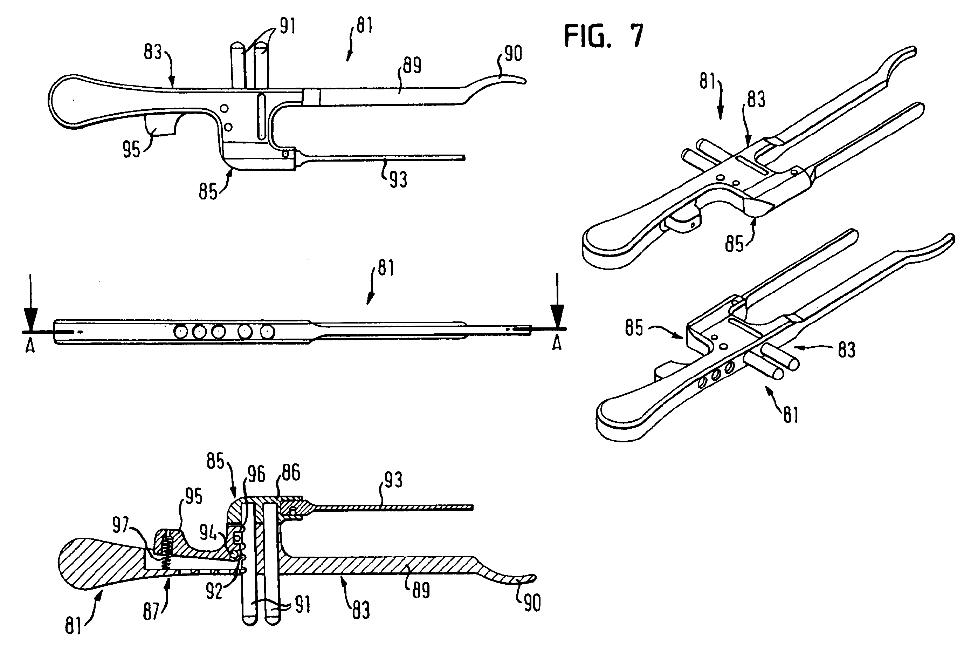

Gemäß Fig. 7 umfasst das Grundteil 85 der Tasteinrichtung 81 einen Träger 86, mit dem einerseits der streifenförmige Kopplungsabschnitt 93 und andererseits die beiden Führungsstifte 91 für den relativ zum Grundteil 85 längs der Führungsstifte 91 verschiebbaren Taster 83 verbunden sind.

Der Taster 83 umfasst den das gekrümmte freie Ende 90 aufweisenden, parallel zum Kopplungsabschnitt 93 verlaufenden Tastarm 89 sowie einen Betätigungsabschnitt 87, an dem ein Betätigungshebel 95 über einen Stift 94 verschwenkbar gelagert ist. Durch eine an dem einen Hebelarm des Betätigungshebels 95 angreifende, sich am Betätigungsabschnitt 87 abstützende Rückstellfeder 97 ist eine Verriegelungsnase 96 des anderen Hebelarms des Betätigungshebels 95 gegen den einen Führungsstift 91 vorgespannt, so dass bei entsprechender Relativstellung zwischen Grundteil 95 und Taster 83 die Verriegelungsnase 96 in an diesem Führungsstift 91 ausgebildete Aussparungen eingreift, wodurch die Relativstellung zwischen Grundteil 85 und Taster 83 fixiert ist. Die Positionen der Rastaussparungen des Führungsstiftes 91 entsprechen den Größen von Tibiaimplantaten eines vorhandenen Implantatsatzes.

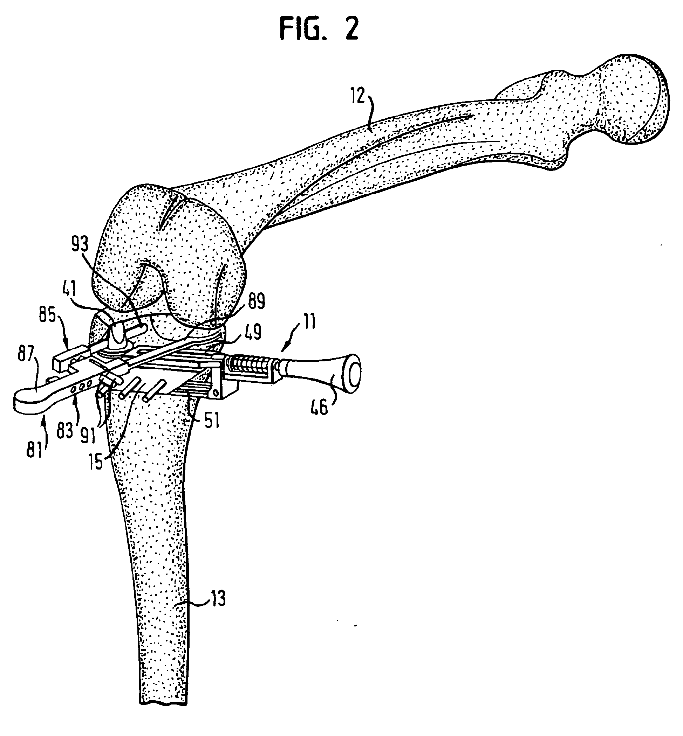

Durch Betätigen des Hebels 95 gegen die Rückstellkraft der Feder 97 kann der die mit der Schnittlehre 11 gekoppelte Tasteinrichtung 81 (vgl. Fig. 2) am Betätigungsabschnitt 87 haltende Operateur die Verrastung des Tasters 93 am Führungsstift 91 lösen und durch Verschieben des Tasters 83 längs der Führungsstifte 91 relativ zum Grundteil 85 alle vorgegebenen Implantatgrößen an der Tasteinrichtung 81 einstellen und auf diese Weise unter Berücksichtigung der anatomischen Gegebenheiten des jeweiligen Knies die für das Knie am besten passende Implantatgröße ermitteln.

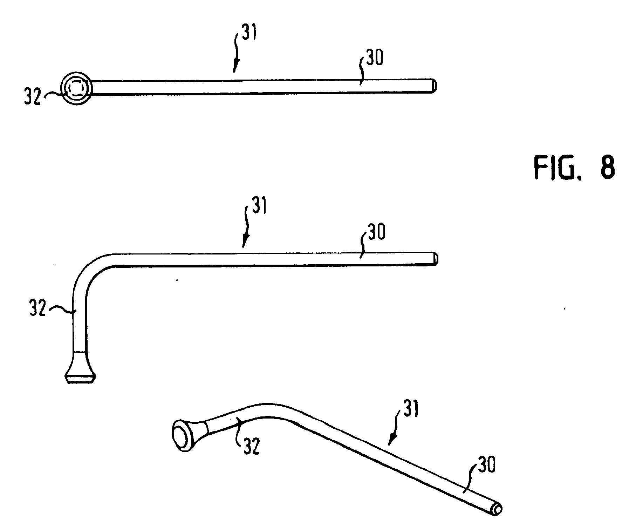

Der in Fig. 8 dargestellte Anschlagstift 31 (vgl. auch Fig. 3 bis 5) umfasst einen geraden Anschlagabschnitt 30, mit dem der Anschlagstift 31 durch den an der Drehtelleranordnung 37 der Schnittlehre 11 ausgebildeten Durchgang 29 hindurch in die zuvor im Knochen ausgebildete Bohrung gesteckt wird, sowie einen um 90° abgewinkelten Halteabschnitt 32, an dem der Operateur den Anschlagstift 31 beim Einstecken in den Knochen und beim Herausziehen aus dem Knochen ergreifen kann. Das freie Ende des Halteabschnitts 32 ist mit einer radialen Erweiterung versehen. Der Anschlagstift besteht aus einer gehärteten Metalllegierung, damit kein Verschleiß bei der seitlichen Begrenzung der Sägeblätter auftritt. Er ist abgewinkelt, damit er nicht mit üblichen geraden Stiften verwechselt werden kann.

### Bezugszeichenliste

- 11 1: Schnittlehre
- 12: Femur
- 13: Tibia
- 15: Basiselement, Schnittblock
- 16: Haltestift
- 17: erster Schlitz
- 19: zweiter Schlitz
- 21: erstes Schneidewerkzeug, erstes Sägeblatt
- 23: zweites Schneidewerkzeug, erstes Sägeblatt
- 25: erste Schnittebene, erste Schnittfläche
- 27: zweite Schnittebene, zweite Schnittfläche
- 29: Führung, Durchgang
- 30: Anschlagabschnitt
- 31: Anschlagelement, Anschlagstift
- 32: Halteabschnitt
- 37: Drehtelleranordnung
- 39: Drehteller
- 41: Führungsabschnitt
- 43: Führungsabschnitt
- 44: Klemmabschnitt
- 45: Betätigungsorgan, Klemmspindel
- 46: Handgriff
- 47: Grundteil
- 49: oberer U-Schenkel
- 51: unterer U-Schenkel
- 53: Klemmhebel
- 54: Stift
- 55: Betätigungsarm
- 56: Gewindeabschnitt
- 57: Klemmarm
- 59: Auflageabschnitt
- 61: Feder
- 62: Abstütz- und Führungsabschnitt
- 81: Tasteinrichtung
- 83: Taster
- 85: Grundteil
- 86: Träger
- 87: Betätigungsabschnitt
- 89: Tastarm
- 90: gekrümmtes freies Ende
- 91: Führungsstift
- 92: Rastaussparung
- 93: Kopplungsabschnitt
- 94: Stift
- 95: Betätigungshebel
- 96: Verriegelungsnase
- 97: Rückstellfeder

## Patentansprüche

1. Vorrichtung zur Festlegung von Knochenschnitten beim Einsetzen von Knieimplantaten, mit wenigstens einer Schnittlehre (11), die mit einem am Knochen im Bereich einer Kondyle fixierten Basiselement (15) koppelbar und an dem Basiselement (15) festsetzbar ist,
**dadurch gekennzeichnet,**
**dass** die Schnittlehre (11) im festgesetzten Zustand jeweils mittels eines Schlitzes (17, 19) für ein Schneidewerkzeug (21, 23) eine erste Schnittebene (25) bezüglich des Basiselements (15) sowie eine zweite Schnittebene (27) bezüglich der ersten Schnittebene (25) definiert,
wobei die Orientierung der zweiten Schnittebene (27) bei mit dem Basiselement (15) gekoppelter Schnittlehre (11) und unter Berücksichtigung der jeweiligen Knieanatomie relativ zur ersten Schnittebene (25) einstellbar ist.

2. Vorrichtung nach Anspruch 1,
**dadurch gekennzeichnet,**
**dass** die erste Schnittebene (25) und die zweite Schnittebene (27) senkrecht zueinander verlaufen.

3. Vorrichtung nach Anspruch 1 oder 2,
**dadurch gekennzeichnet,**
**dass** die Orientierung der zweiten Schnittebene (27) bei relativ zum Basiselement (15) bewegbarer Schnittlehre (11) einstellbar ist, wobei vorzugsweise die Schnittlehre (11) ausschließlich parallel zur ersten Schnittebene (25) und insbesondere ausschließlich geradlinig bewegbar ist.

4. Vorrichtung nach einem der vorhergehenden Ansprüche,
**dadurch gekennzeichnet,**
**dass** die Schnittlehre (11) durch eine am Basiselement (15) ausgebildete Führungsnut am Basiselement (15) zwangsführbar ist.

5. Vorrichtung nach einem der vorhergehenden Ansprüche,
**dadurch gekennzeichnet,**
**dass** die Schnittlehre (11) wenigstens eine Führung (29) für ein Anschlagelement (31) aufweist, das in zumindest einer der Schnittebenen (25, 27) einen den betreffenden Schnitt begrenzenden Anschlag für das Schneidewerkzeug (21, 23) bildet.

6. Vorrichtung nach Anspruch 5,
**dadurch gekennzeichnet,**
**dass** die Führung (29) derart ausgebildet ist, dass das Anschlagelement (31) auf der Schnittlinie der beiden Schnittebenen (25, 27) liegt.

7. Vorrichtung nach Anspruch 5 oder 6,
**dadurch gekennzeichnet,**
**dass** die Führung (29) für das Anschlagelement (31) mit dem die zweite Schnittebene (27) definierenden Schlitz (19) zwangsgekoppelt ist.

8. Vorrichtung nach einem der Ansprüche 5 bis 7,
**dadurch gekennzeichnet,**
**dass** die Führung (29) einen in der Schnittlehre (11) ausgebildeten Durchgang umfasst, durch den das Anschlagelement (31) hindurch steckbar ist.

9. Vorrichtung nach einem der Ansprüche 5 bis 8,
**dadurch gekennzeichnet,**
**dass** das Anschlagelement (31) in Form eines vor dem Ausführen der Knochenschnitte in den Knochen (13) einbringbaren Anschlagstiftes vorgesehen ist.

10. Vorrichtung nach einem der vorhergehenden Ansprüche,
**dadurch gekennzeichnet,**
**dass** zur Berücksichtigung der Knieanatomie eine verstellbare Tasteinrichtung (81) vorgesehen ist, die mit dem die zweite Schnittebene (23) definierenden Schlitz (19) der Schnittlehre (11) zwangskoppelbar und an der durch Verstellen eines Tasters (83) relativ zu einem mit dem Schlitz (19) gekoppelten Grundteil (85) eine Mehrzahl vorgegebener Implantatgrößen einstellbar ist.

11. Vorrichtung nach einem der vorhergehenden Ansprüche,
**dadurch gekennzeichnet,**
**dass** der die erste Schnittebene (25) definierende Schlitz (17) von einem bei mit dem Basiselement (15) gekoppelter Schnittlehre (11) zwischen der Oberseite des Basiselementes (15) und einer dem Basiselement (15) zugewandten Seite der Schnittlehre (11) vorhandenen Zwischenraum gebildet ist.

12. Vorrichtung nach einem der vorhergehenden Ansprüche,
**dadurch gekennzeichnet,**
**dass** die Schnittlehre (11) eine Drehtelleranordnung (37) mit einem an der Schnittlehre (11) drehbar gelagerten Drehteller (39) umfasst, mit dem ein Führungsabschnitt (41) drehfest verbunden ist, in dem der die zweite Schnittebene (27) definierende Schlitz (19) ausgebildet ist, wobei vorzugsweise die Drehachse des Drehtellers (39) senkrecht zur ersten Schnittebene (21) verläuft.

13. Vorrichtung nach Anspruch 12,
**dadurch gekennzeichnet,**
**dass** mit dem Drehteller (39) ein weiterer Führungsabschnitt (43) drehfest verbunden ist, der als die Führung (29) für das Anschlagelement (31) ausgebildet ist.

14. Vorrichtung nach einem der vorhergehenden Ansprüche,
**dadurch gekennzeichnet,**
**dass** die Schnittlehre (11) eine Klemmeinrichtung mit einem Betätigungsorgan (45) aufweist, mittels welchem gleichzeitig die Schnittlehre (11) relativ zum Basiselement (15) und die Orientierung der zweiten Schnittebene (27) relativ zur ersten Schnittebene (25) festsetzbar ist.

15. Vorrichtung nach einem der vorhergehenden Ansprüche,
**dadurch gekennzeichnet,**
**dass** die Schnittlehre (11) ein U-förmig ausgebildetes, auf das Basiselement (15) aufschiebbares Grundteil (47) mit parallel zur ersten Schnittebene (25) verlaufenden U-Schenkeln (49, 51), einen um eine parallel zur ersten Schnittebene (25) und senkrecht zu den U-Schenkeln (49, 51) des Grundteils (47) verlaufende Achse schwenkbar am Grundteil (47) gelagerten Klemmhebel (53) und eine sich parallel zu den U-Schenkeln (49, 51) des Grundteils (47) erstreckende, mit dem Klemmhebel (53) über ein Gewinde zusammenwirkende Klemmspindel (45) umfasst,
wobei durch eine Drehbetätigung der Klemmspindel (45) gleichzeitig eine am oberen U-Schenkel (49) des Grundteils (47) um eine senkrecht zur ersten Schnittebene (25) verlaufende Achse drehbar gelagerte, den die zweite Schnittebene (27) definierenden Schlitz (19) aufweisende Drehtelleranordnung (37) festsetzbar und
der Klemmhebel (53) relativ zum Grundteil (47) in Klemmeingriff mit dem Basiselement (15) schwenkbar ist.

16. Vorrichtung nach Anspruch 15,
**dadurch gekennzeichnet,**
**dass** ein freier Endbereich der Klemmspindel (45) zum Festsetzen der Drehtelleranordnung (37) ausgebildet und durch die Drehbetätigung der Klemmspindel (45) in Klemmeingriff mit einem äußeren Randbereich der Drehtelleranordnung (37) bringbar ist.

17. Vorrichtung nach Anspruch 15 oder 16,
**dadurch gekennzeichnet,**
**dass** der Klemmhebel (53) einen Betätigungsarm (55) und einen Klemmarm (57) umfasst, wobei die Klemmspindel (45) mit dem Betätigungsarm (55) zusammenwirkt und der Klemmarm (57) zum Festklemmen des zwischen den beiden U-Schenkeln (49, 51) des Grundteils (47) befindlichen Basiselements (15) durch die Drehbetätigung der Klemmspindel (45) über den Betätigungsarm (55) in den Bereich zwischen den beiden U-Schenkel (49, 51) hinein und gegen das Basiselement (15) schwenkbar ist.

18. Vorrichtung nach einem der Ansprüche 15 bis 17,
**dadurch gekennzeichnet,**
**dass** zusammenwirkende Gewindeabschnitte der Klemmspindel (45) und des Klemmhebels (53) durch eine zwischen einen spindelfesten Auflageabschnitt (59) und den Betätigungsarm (55) des Klemmhebels (53) gespannte Feder (61) spielfrei gehalten sind.

19. Vorrichtung nach einem der Ansprüche 15 bis 18,
**dadurch gekennzeichnet,**
**dass** der Zwischenraum zwischen den beiden U-Schenkeln (49, 51) des Grundteils (47) auf die Höhe des Basiselements (15) abgestimmt und für ein Basiselement (15) mit einer Höhe von etwa 12 mm ausgelegt ist.

## Claims

1. An apparatus for the fixing of the position of bone cuts for the insertion of knee implants, comprising at least one cutting jig (11) which can be coupled to a base element (15) fixed to the bone in the region of a condyle and can be fixed to the base element (15),
**characterized in that** the cutting jig (11) defines, in the fixed state and in each case by means of a slot (17, 19) for a cutting tool (21, 23), a first cutting plane (25) with respect to the base element (15) and a second cutting plane (27) with respect to the first cutting plane (25), wherein the orientation of the second cutting plane (27) is adjustable relative to the first cutting plane (25) when the cutting jig (11) is coupled to the base element (15) and while taking the respective knee anatomy into account.

2. An apparatus in accordance with claim 1, **characterized in that** the first cutting plane (25) and the second cutting plane (27) extend perpendicular to one another.

3. An apparatus in accordance with claim 1 or claim 2, **characterized in that** the orientation of the second cutting plane (27) is adjustable with the cutting jig (11) movable relative to the base element (15), with the cutting jig (11) preferably being movable only parallel to the first cutting plane (25) and in particular only in a straight line.

4. An apparatus in accordance with any one of the preceding claims, **characterized in that** the cutting jig (11) is compulsorily guided at the base element (15) by a guide groove formed at the base element (15).

5. An apparatus in accordance with any one of the preceding claims, **characterized in that** the cutting jig (11) has at least one guide (29) for an abutment element (31) which, in at least one of the cutting planes (25, 27), forms an abutment for the cutting tool (21, 23) restricting the respective cut.

6. An apparatus in accordance with claim 5, **characterized in that** the guide (29) is made such that the abutment element (31) lies on the line of intersection (33) of the two cutting planes (25, 27).

7. An apparatus in accordance with claim 5 or claim 6, **characterized in that** the guide (29) for the abutment element (31) is compulsorily coupled to the slot (19) defining the second cutting plane (27).

8. An apparatus in accordance with any one of the claims 5 to 7, **characterized in that** the guide (29) includes a passage formed in the cutting jig (11) through which the abutment element (31) can be inserted.

9. An apparatus in accordance with any one of the claims 5 to 8, **characterized in that** the abutment element (31) is provided in the form of an abutment pin which can be introduced into the bone (13) before the carrying out of the bone cuts.

10. An apparatus in accordance with any one of the preceding claims, **characterized in that** an adjustable probe device (81) is provided to take the knee anatomy into account and can be compulsorily coupled to the slot (19) of the cutting jig (11) defining the second cutting plane (23), with it being possible to set a plurality of pre-determined implant sizes by adjusting a probe (83) relative to a base part (85) coupled to the slot (19).

11. An apparatus in accordance with any one of the preceding claims, **characterized in that** the slot (17) defining the first cutting plane (25) is formed by an intermediate space present between the upper side of the base element (15) and a side of the cutting jig (11) facing the base element (15) when the cutting jig (11) is coupled to the base element (15).

12. An apparatus in accordance with any one of the preceding claims, **characterized in that** the cutting jig (11) includes a turntable arrangement (37) with a turntable (39) which is rotatably supported at the cutting jig (11) and with which a guide section (41) is rotatably fixedly connected in which the slot (19) defining the second cutting plane (27) is formed, wherein the rotational axis of the turntable (39) preferably extends perpendicular to the first cutting plane (21).

13. An apparatus in accordance with claim 12, **characterized in that** a further guide section (43) is rotatably fixedly connected to the turntable (39) and is formed as a guide (29) for the abutment element (31).

14. An apparatus in accordance with any one of the preceding claims, **characterized in that** the cutting jig (11) has a clamping device with an actuating member (45) by means of which the cutting jig (11) can be fixed relative to the base element (15) and simultaneously the orientation of the second cutting plane (27) can be fixed relative to the first cutting plane (25).

15. An apparatus in accordance with any one of the preceding claims, **characterized in that** the cutting jig (11) includes a U-shaped base part (47) which can be pushed onto the base element (15) and having U-limbs (49, 51) extending parallel to the first cutting plane (25), a clamping lever (53) pivotably supported at the base part (47) about an axis extending parallel to the first cutting plane (25) and perpendicular to the U limbs (49, 51) of the base part (47) and a clamping spindle (45) extending parallel to the U limbs (49, 51) of the base part (47) and cooperating with the clamping lever (53) via a thread, wherein a turntable arrangement (37) rotatably supported at the upper U limb (49) of the base part (47) about an axis extending perpendicular to the first cutting plane (25) and having the slot (19) defining the second cutting plane (27) can be fixed by a rotational actuation of the clamping spindle (45) and simultaneously the clamping lever (53) is pivotable relative to the base part (47) into clamping engagement with the base element (15).

16. An apparatus in accordance with claim 15, **characterized in that** a free end region of the clamping spindle (45) is made for the fixing of the turntable arrangement (37) and can be brought into clamping engagement with an outer rim region of the turntable arrangement (37) by the rotational actuation of the clamping spindle (45).

17. An apparatus in accordance with claim 15 or claim 16, **characterized in that** the clamping lever (53) includes an actuation arm (55) and a clamping arm (57), with the clamping spindle (45) cooperating with the actuating arm (55) and the clamping arm (57) being pivotable, for the clamping tight of the base element (15) located between the two U limbs (49, 51) of the base part (47), by rotational actuation of the clamping spindle (45) via the actuating arm (55) into the region between the two U limbs (49, 51) and toward the base element (15).

18. An apparatus in accordance with any one of the claims 15 to 17, **characterized in that** cooperating threaded sections of the clamping spindle (45) and of the clamping lever (53) are held free of clearance by a spring (61) clamped between a contact section (59) fixed with respect to the spindle and the actuation arm (55) of the clamping lever (53).

19. An apparatus in accordance with any one of the claims 15 to 18, **characterized in that** the intermediate space between the two U limbs (49, 51) of the base part (47) is matched to the height of the base element (15) and is designed for a base element (15) with a height of approximately 12 mm.

## Revendications

1. Dispositif de détermination de coupes osseuses lors de la mise en place d'implants de genou, comportant au moins un gabarit de coupe (11) susceptible d'être couplé à un élément de base (15) fixé à l'os dans la zone d'un condyle et d'être immobilisé sur l'élément de base (15),
**caractérisé en ce que**
dans l'état immobilisé, le gabarit de coupe (11) définit, au moyen d'une fente respective (17, 19), un premier plan de coupe (25) par rapport à l'élément de base (15) ainsi qu'un second plan de coupe (27) par rapport au premier plan de coupe (25) pour un outil de coupe (21, 23), l'orientation du second plan de coupe (27) étant réglable par rapport au premier plan de coupe (25), le gabarit de coupe (11) étant couplé à l'élément de base (15), tout en tenant compte de l'anatomie de genou respective.

2. Dispositif selon la revendication 1,
**caractérisé en ce que**
le premier plan de coupe (25) et le second plan de coupe (27) s'étendent perpendiculairement l'un à l'autre.

3. Dispositif selon la revendication 1 ou 2,
**caractérisé en ce que**
l'orientation du second plan de coupe (27) est réglable avec le gabarit de coupe (11) mobile par rapport à l'élément de base (15), le gabarit de coupe (11) étant mobile de préférence exclusivement parallèlement au premier plan de coupe (25) et en particulier exclusivement en ligne droite.

4. Dispositif selon l'une des revendications précédentes,
**caractérisé en ce que**
le gabarit de coupe (11) est susceptible d'être guidé à force sur l'élément de base (15) par une gorge de guidage ménagée dans l'élément de base (15).

5. Dispositif selon l'une des revendications précédentes,
**caractérisé en ce que**
le gabarit de coupe (11) comprend au moins un guidage (29) pour un élément de butée (31) qui forme pour l'outil de coupe (21, 23) une butée qui limite la coupe concernée dans l'un au moins des plans de coupe (25, 27).

6. Dispositif selon la revendication 5,
**caractérisé en ce que**
le guidage (29) est réalisé de telle sorte que l'élément de butée (31) se trouve sur la ligne de coupe des deux plans de coupe (25, 27).

7. Dispositif selon la revendication 5 ou 6,
**caractérisé en ce que**
le guidage (29) pour l'élément de butée (31) est couplé à force avec la fente (19) qui définit le second plan de coupe (27).

8. Dispositif selon l'une des revendications 5 à 7,
**caractérisé en ce que**
le guidage (29) présente un passage ménagé dans le gabarit de coupe (11), à travers lequel peut être enfiché l'élément de butée (31).

9. Dispositif selon l'une des revendications 5 à 8,
**caractérisé en ce que**
l'élément de butée (31) est prévu sous la forme d'une tige de butée susceptible d'être introduite dans l'os (13) avant d'exécuter les coupes osseuses.

10. Dispositif selon l'une des revendications précédentes,
**caractérisé en ce que**
pour tenir compte de l'anatomie du genou, il est prévu un dispositif de palpage déplaçable (81) qui est susceptible d'être couplé à force à la fente (19) du gabarit de coupe (11) définissant le second plan de coupe (23) et sur lequel une pluralité de tailles d'implant prédéterminées peuvent être réglées par déplacement d'un palpeur (83) par rapport à une partie de base (85) couplée à la fente (19).

11. Dispositif selon l'une des revendications précédentes,
**caractérisé en ce que**
la fente (17) définissant le premier plan de coupe (25) est formée par un intervalle existant entre le côté supérieur de l'élément de base (15) et un côté du gabarit de coupe (11) tourné vers l'élément de base (15), le gabarit de coupe (11) étant couplé à l'élément de base (15).

12. Dispositif selon l'une des revendications précédentes,
**caractérisé en ce que**
le gabarit de coupe (11) comprend un agencement de plateau tournant (37) pourvu d'un plateau tournant (39) monté mobile en rotation sur le gabarit de coupe (11) et auquel est relié solidairement en rotation un tronçon de guidage (41) dans lequel est ménagée la fente (19) définissant le second plan de coupe (27), l'axe de rotation du plateau tournant (39) s'étendant de préférence perpendiculairement au premier plan de coupe (21).

13. Dispositif selon la revendication 12,
**caractérisé en ce que**
au plateau tournant (39) est relié solidairement en rotation un autre tronçon de guidage (43) qui est réalisé sous forme de guidage (29) pour l'élément de butée (31).

14. Dispositif selon l'une des revendications précédentes,
**caractérisé en ce que**
le gabarit de coupe (11) comprend un dispositif de serrage pourvu d'un organe d'actionnement (45) au moyen duquel le gabarit de coupe (11) est susceptible d'être immobilisé par rapport à l'élément de base (15) et simultanément l'orientation du second plan de coupe (27) peut être déterminée par rapport au premier plan de coupe (25).

15. Dispositif selon l'une des revendications précédentes,
**caractérisé en ce que**
le gabarit de coupe (11) comprend une partie de base (47) réalisée en forme de U, susceptible d'être enfilée sur l'élément de base (15) et pourvue de branches en U (49, 51) parallèles au premier plan de coupe (25), un levier de serrage (53) monté sur la partie de base (47) avec faculté de pivotement autour d'un axe parallèle au premier plan de coupe (25) et perpendiculaire aux branches en U (49, 51) de la partie de base (47), et une broche de serrage (45) parallèle aux branches en U (49, 51) de la partie de base (47) et coopérant avec le levier de serrage (53) via un pas de vis,
dans lequel, par un actionnement en rotation de la broche de serrage (45), un agencement de plateau tournant (37) monté mobile en rotation autour d'un axe perpendiculaire au premier plan de coupe (25) sur la branche en U supérieure (49) de la partie de base (47) et présentant la fente (19) définissant le second plan de coupe (27) est susceptible d'être immobilisé et simultanément le levier de serrage (53) est mobile en pivotement par rapport à la partie de base (47) en étant en engagement serré avec l'élément de base (15).

16. Dispositif selon la revendication 15,
**caractérisé en ce que**
il est prévu une zone d'extrémité libre de la broche de serrage (45) pour immobiliser l'agencement de plateau tournant (37), qui est susceptible d'être amenée en engagement serré avec une zone de bord extérieure de l'agencement de plateau tournant (37) par un actionnement en rotation de la broche de serrage (45).

17. Dispositif selon la revendication 15 ou 16,
**caractérisé en ce que**
le levier de serrage (53) comprend un bras d'actionnement (55) et un bras de serrage (57), la broche de serrage (45) coopérant avec le bras d'actionnement (55), et pour serrer l'élément de base (15) situé entre les deux branches en U (49, 51) de la partie de base (47), le bras de serrage (57) est susceptible de pivoter jusque dans la zone entre les deux branches en U (49, 51) et contre l'élément de base (15) via le bras d'actionnement (55) par l'actionnement en rotation de la broche de serrage (45).

18. Dispositif selon l'une des revendications 15 à 17,
**caractérisé en ce que**
des tronçons à pas de vis coopérant de la broche de serrage (45) et du levier de serrage (53) sont maintenus sans jeu par un ressort (61) bandé entre un tronçon de support (59) solidaire de la broche et le bras d'actionnement (55) du levier de serrage (53).

19. Dispositif selon l'une des revendications 15 à 18,
**caractérisé en ce que**
l'intervalle entre les deux branches en U (49, 51) de la partie de base (47) est adapté à la hauteur de l'élément de base (15) et est conçu pour un élément de base (15) d'une hauteur d'environ 12 mm.
